# EUROPEAN PATENT SPECIFICATION

(11) **EP 1 949 900 B1**
(45) Date of publication and mention of the grant of the patent: **14.09.2016**
(21) Application number: 08075015.1
(22) Date of filing: 07.01.2008
(51) Int. Cl.: A61K 31/385, A61K 9/00

(54) **Controlled release solid formulation for oral administration as single dose sachet and method of preparation thereof**
Feste Formulierung mit kontrollierter Freisetzung zur oralen Verabreichung als Einzeldosispackung und Herstellungsverfahren dafür
Formulation solide à libération contrôlée pour l'administration orale en tant que sachet monodose et son procédé de préparation

(30) Priority: 09.01.2007 IT MI20070022
(43) Date of publication of application: 30.07.2008
(73) Proprietor: I.P.S. International Products & Services S.r.l., 20097 S. Donato Milanese (IT)
(72) Inventor: Montano, Francesco, 20097 S. Donato Milanese (IT); Pasotti, Gino, 20097 S. Donato Milanese (IT); Spalla, Luciano, 20097 S. Donato Milanese (IT); Legrottaglie, Maria Teresa, 20097 S. Donato Milanese (IT)
(74) Representative: Riccardi, Sergio

(56) References cited:
- EP-A2- 0 310 814
- WO-A1-01/66094
- WO-A1-99/61004
- WO-A2-2004/056336
- WO-A2-2007/010501

## Description

The present invention relates to a formulation of controlled release solid products for oral administration as single dose sachets as well as a method of their preparation.

Solid products such as medicaments and food supplements for oral administration with controlled release, in the form of granules, pellets or tablets are well known. Their success is due to the fact that the active substance is released to the organism in a controlled, gradual, sustained or retarded way, thus avoiding plural administration during the day and other undesired effects.

With regard to pellets, inert granules are covered with a layer of active substance and then an outer membrane causing the controlled and/or delayed release, and subsequently a dosage of these pellets is filled into hard gelatine capsules, with the further advantage in comparison with other administration forms such as lozenges and tablets, to be spreaded homogeneously in many (some hundred) minidoses on a wide length of the gastro-intestinal tract, thus minimizing the active substance concentration of each fraction of the total dose, with consequent reduction of side effects such as gastric irritation and poor absorption by the organism.

However this technology, like the preparation of tablets, is subject to the maximum admissible dosage for each active substance, due to the maximum size of capsule or tablet that a person is able to swallow.

In view of this, it is difficult or even impossible to formulate in the form of controlled release administration substances requiring a high dosage, that in such a form would require the administration of two or more capsules or tablets (an impractical and commercially unacceptable solution), or intended for geriatic or pediatric use, where administration of capsules or tablets over a given size results impossible for swallowing difficulties of these users.

In both the above mentioned cases the problem may be solved by administering the product in single dose sachets or bags, allowing to take greater quantities of active substance in retard form either pouring directly in the user's mouth the properly sweetened and/or flavoured contents, or pouring said contents into a small quantity of liquid (water, milk and the like) and drinking it after a short agitation, as well as to add other active ingredients, in any administration form. In both cases the pellets should be swallowed without chewing and reach intact the stomach or the bowels, then starting the controlled or retarded release of the active substance.

Therefore it is apparent that for this purpose the pellets must be much smaller that the conventional ones filled in the hard gelatine capsules, so as to flow smoothly through the oral cavity, the pharynx and the oesophagus thus reaching intact the stomach.

Document EP 0 310 814 A2 discloses controlled release formulations specifically adapted to control release of tetracycline compounds, where the spherical granules are adapted only to prepare capsules or tablets in view of their final size not suitable to prepare minipellets for single dose sachets.

Document WO 2007/010501 A1 discloses a pharmaceutical composition comprising a combination of beta blocker and an ACE inhibitor, where the final administration form consists only of capsules and tablets even multilayered, not adapted to be prepared for single dose sachets. No taste masking ingredients are disclosed.

Document WO 01/66094 A1 discloses controlled release oral solid forms containing mesalazine, namely beadlets only adapted for capsules of rigid gelatine, because under a specific size the beadlet titre would be too low, thus requiring a capsule too big to be swallowed.

Document WO 99/61004 A1 discloses a controlled release formulation of lipoic acid, specifically adapted to protect lipoic acid from chemical degradation in the gastro-intestinal tract. The disclosed administration forms always comprise tablets, caplets, capsules of different types, but there is no mention of a formulation allowing to prepare lipoic acid in powder form to be prepared is monodose sachets.

Document WO 2004/056336 discloses controlled release, multiple unit drug delivery systems more particularly carvedilol, to be formulated in capsules or tablets. The description states that it is believed that the formulation might be filled into sachets, but there is no indication whatsoever of how to prepare such a powder in view of the parameters used, totally unsuitable for such use.

Unlike all these prior art citations, the present invention relates to a formulation that can be used to produce both pharmaceutical substances and food supplements, comprising the unique combination of the following features: oral administration form of dispersible or orosoluble sachets; modified release, namely after unaltered passage in the oro-pharyngeal and oesophageal tract, continuous and regular release in the gastro-intestinal tract for a period of 6-10 hours, independently from the solubility of the active substance; particles of the active substance with a size under 710 µm avoiding the sand effect in the oral cavity; proper masking of unpleasant odor and/or taste.

The present invention brilliantly solves this problem, allowing to obtain pellets of very little size, comprising the inert microgranule covered with the active substance, the controlled release membrane and possibly gelifying excipients, to obtain a homogeneous suspension of pellets, when the sachet should be poured into a liquid.

Indeed the contents of the single dose sachets should be normally sweetened and flavoured to be palatable, since both the drugs and the food supplements have unpleasant smell and taste, which are considerably masked by the controlled release membrane and the addition of said flavourings, sweeteners and possibly gelifiers, that must be added in a solid form, because the liquid form of these substances would cause the contents of the sachet to be packed, thus hindering the smooth flow of the pellets on swallowing.

Therefore the formulation of the present invention is prepared with the normal equipment used for producing controlled release granules, starting from inert granules with size between 250 and 600 µm, that are covered with active substances with a particle size between 1 and 200 µm.

Above and under these ranges it is not possible to obtain the controlled release. One of the very thin known membranes providing for the controlled release is then applied on the granules.

At this point the above mentioned flavouring, sweetening and possibly gelifying substances are added to the pellets, in a solid form of powder or granulate. However, as these substances in their solid form have a different specific gravity and particle size relative to the pellets, with the risk that the product becomes unblended on preparing the sachets, this problem is solved by covering the pellets with a mixture of sweeteners and flavourings dissolved in a proper solvent, that will be evaporated once said substances adhered stably on the pellet surface, sticking to the surface without causing modification of the controlled release membrane and consequently changes of the release time and/or amount of the active substance contained in the pellets.

The same problem may alternatively be solved by making a special kind of pellets containing the sweetening and flavouring substances only, with immediate release of their contents, having the same size and specific gravity of the pellets containing the active substance, in a quantity adapted for the mechanical filling of the sachet, avoiding the unblending risk and warranting the exact dosage of active substance for each sachet. In this way, when administering the sachet contents, the pellets containing said sweetening and flavouring substances release immediately their contents, so that the user's taste buds perceive only these substances, while the pellets containing the active substance flow to the oesophagus without any problem.

Another advantage of the present invention is that the technology used to obtain the above described controlled release pellets, allows not to exceed a temperature of 35°C during the entire preparation process, this being very important for ingredients with low melting or polymerisation point, such as the thioptic acid (known also as alpha lipoic acid), whose chemico-physical characteristics would be negatively altered by exposure to higher temperatures, like it normally happens with the normal technologies for producing lozenges or tablets.

As an illustrative and non limiting example, the technique for preparing a formulation of controlled release pellets of thioptic acid is now given hereinafter, a food supplement that for its very short half life (26 minutes) is particularly suitable for a controlled release administration form.

With the conventional granules administered in hard gelatine capsules of the biggest acceptable size, it is possible to reach a maximum dosage of 400 mg per capsule. With the pellets prepared according to the present invention, there is no difficulty to prepare a single dose sachet containing the desired dosage of 800 mg, with controlled release pellets, flavoured to mask the task of thioptic acid, following the method hereinbelow illustrated.

Starting from inert granules of size between 250 and 600 µm, covered with the active substance and a suitable membrane adapted to give the desired release pattern, retard pellets with controlled release of thioptic acid are obtained, having a particle size between 425 µm and 710 µm and a titer between 200 and 400 mg/g according to the desired final dosage. The in vitro release rates, measured with the method and apparatus officially described in the current edition of the European Pharmacopoeia, are comprised in the following percentage ranges:

| | |
|---|---|
| 1^{st} hour | 15-45% |
| 2^{nd} hour | 45-70% |
| 4^{th} hour | 65-85% |
| 8^{th} hour | ≥ 90% |

Said pellets are then flavoured, without changing the above indicated release characteristics, so as to remove the spicy taste of thioptic acid. Suspensions and solutions are prepared by using water as a solvent, to avoid an alteration of membrane when using solvents such as ethyl alcohol, acetone, methanol and so on. The application of flavouring agents may be carried out in suspension or powder according to the following methods.

### Method 1 - Application of flavouring agents in suspension.

Transfer 1960 g of retard pellets in a 10 Lt. pan.

Start rotation of pan and using a spraying gun and a peristaltic pump, spray a suspension of flavourings and binder with the following composition:

| | |
|---|---|
| Powdered mandarin flavour | 7.0 g |
| Powdered grapefruit flavour | 7.0 g |
| Citric acid | 3.5 g |
| Acetosulphame K | 0.21 g |
| Povidone K 30 | 4.0 g |
| Water | 26.5 g |

At the end, let the product dry at room temperature and sprinkle with 10 g of talc to remove the electrostatic charges.

### Method 2 - Application of flavouring agents in powder form.

Transfer 1960 g of retard pellets in a 10 Lt. pan.

Start rotation of pan and using a spraying gun and a peristaltic pump spray the following solution:

| | |
|---|---|
| Citric acid | 7.0 g |
| Acetosulphame | 0.42 g |
| Povidone K 30 | 3.0 g |
| Water | 18.0 g |

Apply to the product 14 g of powdered mandarin flavour and 14 g of powdered grapefruit flavour. Let the product dry at room temperature and sprinkle with 10 g of talc to remove the electrostatic charges.

It has be noted that modifications, variations, additions and the like may be made to the invention, as suggested by the practice of preparing formulations of the various active substances, without departing however from the objects of the invention nor from its protection scope, as defined in the appended claims.

## Claims

1. A controlled release formulation of temperature sensible lipoic acid for oral administration, comprising pellets, consisting of inert particles coated with a lipoic acid composition in turn covered with a controlled release membrane, and further covered with a mixture of solid sweetening and flavouring agents, dissolved in a suitable solvent to be evaporated after adhesion of said agents on the pellet surface, without modifying the characteristics of the controlled release membrane, said pellets being formulated into a sachet primarily used for direct oral administration to people who cannot swallow tablets or capsules easily, keeping the dimensional final size of the pellets below 710 microns.

2. The formulation of Claim 1, wherein a second population of immediate release pellets is added, with the same size and specific gravity as the controlled release pellets, to avoid their segregation in the sachet, so as to mask the taste and/or smell of the active ingredient when swallowing the product.

3. The formulation of Claim 1, wherein gelifying excipients are further added to enhance a homogeneous suspension of pellets when the sachet contents is poured into a liquid for its administration.

4. A method for preparing the formulation according to the preceding claims, comprising the following steps:
- preparing inert cores with a size between 250 and 600 microns;
- covering the cores with an active ingredient having a size between 1 and 200 microns;
- applying on the covered core a membrane adapted to give the desired controlled release, obtaining pellets adapted for direct administration, with a final granule size below 710 microns;
- further covering the pellets with a flavouring and/or sweetening mixture; and
- preparing the intended dosage of active substance contained in said pellets, in the form of single dose sachets.

5. The method of Claim 4, wherein the flavouring mixture is applied by spraying a suspension of flavouring agents and binders on the pellets.

6. The method of Claim 4, wherein the flavouring mixture is applied in powdered form on the pellets previously sprayed with a binding suspension.

7. The method according to Claim 4, wherein immediate release pellets containing sweetening and/or flavouring agents, with size and specific gravity equal to those of the controlled release pellets, to avoid their segregation in the sachet, are added in the single dose sachet.

8. The method according to one or more of Claims 4-7, wherein the entire production process is carried out at a temperature not higher than 35°C, avoiding exposure of the ingredients to higher temperatures that may alter negatively their chemico-physical characteristics.

## Patentansprüche

1. Formulierung mit kontrollierten Freisetzung von temperaturempfindlicher Liponsäure (Thioctsäure) zur oralen Verabreichung, umfassend Pellets, die aus inerten mit einer Zusammensetzung von Liponsäure beschichteten Teilchen bestehen, die wiederum mit einer Mischung von festen Enthärtungsmitteln und Geschmackzusätzen beschichtet werden, die in einem geeigneten Lösungsmittel aufgelöst werden, wobei das Lösungsmittel nach Haften der Mischung an die Pelletsfläche ohne Änderung der Merkmale der Membran mit kontrollierter Freisetzung eingedampft wird, worin die Pellets in eine Packung formuliert werden, die für eine unmittelbare orale Verabreichung hauptsächlich an Leute, die Tabletten bzw. Kapseln kaum schluckern können, verwendet wird, wobei die endgültige Größe der Pellets unter 710 Mikron beibehalten wird.

2. Formulierung nach Anspruch 1, worin eine zweite Population Pellets mit sofortiger Freisetzung mit der selben Größe und Wichte als die Pellets mit kontrollierter Freisetzung zugesetzt wird, um ihre Entmischung in der Packung zur verhindern, und den Geschmack und/oder den Geruch des Wirkstoffs beim Schlucken des Produkts zu maskieren.

3. Formulierung nach Anspruch 1, worin Geliermittel außerdem zugesetzt werden, um eine homogene Suspension der Pellets zu verstärken, als der Inhalt der Packung in eine Flüssigkeit zu seiner Verabreichung gegossen wird.

4. Herstellungsverfahren für die Formulierung nach den vorhergegangenen Ansprüche mit den folgenden Schritten:
- Vorbereitung inerter Kerne mit einer Größe zwischen 250 und 600 Mikrons;
- Beschichtung der Kerne durch einen Wirkstoff mit einer Größe zwischen 1 und 200 Mikrons;
- Anlegen auf dem beschichteten Kern einer geeigneten Membran für die gewünschte kontrollierte Freisetzung, um Pellets für die unmittelbare Verabreichung mit einer endgültigen Kerngrösse unter 710 Mikrons zu erhalten;
- Weitere Beschichtung der Pellets mit einer Mischung von Enthärtungsmitteln und/oder Geschmackzusätzen; und
- Vorbereitung der geplanten Dosierung des in den Pellets enthaltenen Wirkstoffs als Einzeldosispackung.

5. Verfahren nach Anspruch 4, worin die Geschmacksmischung an den Pellets derart angelegt wird, dass eine Suspension von Geschmackzusätzen und Bindemitteln auf die Pellets besprüht wird.

6. Verfahren nach Anspruch 4, worin die Geschmacksmischung in pulverisierter Form auf die Pellets angelegt wird, worin die Pellets mit einer bindenden Suspension vorher besprüht werden.

7. Verfahren nach Anspruch 4, worin Pellets, mit sofortiger Freisetzung und der selben Größe und Wichte als die Pellets mit kontrollierter Freisetzung, in der Einzeldosispackung zugesetzt werden, um ihre Entmischung in der Packung zu verhindern.

8. Verfahren nach einem bzw. mehreren Ansprüchen 4-7, worin das ganze Fertigungsverfahren bei einer Temperatur nicht höher als 35°C ausgeführt wird, indem die Belastung der Bestandteile an höheren Temperaturen, die ihre chemischphysikalischen Eigenschaften nachteilig verändern können, vermieden wird.

## Revendications

1. Formulation à libération contrôlée de l'acide lipoïque pour l'administration orale, comprenant pellets formés par particules inertes revêtues par une composition d'acide lipoïque, à son tour enrobée par une membrane à libération contrôlée et encore enrobée par un mélange d'agents solides adoucissants et aromatisants, dissous dans un solvant approprié évaporable après adhérence des agents sur la surface du pellet, sans modification des caractéristiques de la membrane à libération contrôlée, les dits pellets étant formulés dans un sachet utilisé premièrement pour l'administration orale directe aux gens ayant des difficultés pour avaler comprimés ou capsules, en gardant la dimension finale des pellets au-dessous de 710 microns.

2. Formulation selon la revendication 1, dont une deuxième population des pellets à libération immédiate est ajoutée, avec la même dimension et le même poids spécifique des pellets à libération contrôlée, pour éviter leur ségrégation dans le sachet et masquer la saveur et/ou odeur de la substance active en avalant le produit.

3. Formulation selon la revendication 1, dont des excipients gélifiants sont en outre ajoutés pour améliorer une suspension homogène des pellets, lorsque le contenu du sachet est versé dans un liquide pour sa administration.

4. Procédé de préparation de la formulation selon les revendications précédentes, comprenant les étapes suivantes :
- Préparation des noyaux inertes avec une dimension entre 250 et 600 microns ;
- Revêtir les noyaux par une substance active avec une dimension entre 1 et 200 microns ;
- Appliquer sur le noyau revêtu une membrane appropriée pour donner la libération contrôlée voulue, en obtenant des pellets aptes pour une administration directe, avec une dimension finale du granule au-dessous de 710 microns ;
- Revêtir ultérieurement les pellets par un mélange d'agents adoucissants et aromatisants ; et
- Préparation du dosage prévu de la substance active contenue dans les pellets, sous la forme de sachet monodose.

5. Procédé selon la revendication 4, dont le mélange aromatisant est appliqué par aspersion d'une suspension d'agents aromatisants et liants sur les pellets.

6. Procédé selon la revendication 4, dont le mélange aromatisant est appliqué en forme pulvérisée sur les pellets précédemment aspergés par une suspension liante.

7. Procédé selon la revendication 4, dont dans le sachet monodose on adjoute des pellets à libération immédiate contenant des agents adoucissants et aromatisants, avec la même dimension et le même poids spécifique des pellets à libération contrôlée, pour éviter leur ségrégation dans le sachet.

8. Procédé selon une ou plusieurs revendications 4 - 7, dont le procédé entier de préparation est effectué à une température non dépassant 35°C, en évitant l'exposition des ingrédients aux températures plus élevées pouvant altérer négativement leur caractéristiques chimico-physiques.
